(19) Europäisches Patentamt
European Patent Office
Office européen des brevets

(11) **EP 3 821 883 B1**

(12) **EUROPEAN PATENT SPECIFICATION**

(45) Date of publication and mention
of the grant of the patent:
**17.01.2024 Bulletin 2024/03**

(21) Application number: **19834887.2**

(22) Date of filing: **10.07.2019**

(51) International Patent Classification (IPC):
*A61K 9/70* (2006.01)    *A61K 47/10* (2017.01)
*A61K 47/14* (2017.01)    *A61K 47/18* (2017.01)
*A61K 47/32* (2006.01)    *A61K 47/34* (2017.01)

(52) Cooperative Patent Classification (CPC):
**A61K 9/70; A61K 47/10; A61K 47/14; A61K 47/18;
A61K 47/32; A61K 47/34**

(86) International application number:
**PCT/JP2019/027375**

(87) International publication number:
**WO 2020/013241 (16.01.2020 Gazette 2020/03)**

(54) **ADHESIVE COMPOSITION AND ADHESIVE PATCH**

KLEBSTOFFZUSAMMENSETZUNG UND KLEBEPFLASTER

COMPOSITION ADHÉSIVE ET PATCH ADHÉSIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.07.2018   JP 2018132158
12.07.2018   JP 2018132159**

(43) Date of publication of application:
**19.05.2021 Bulletin 2021/20**

(73) Proprietor: **SEKISUI CHEMICAL CO., LTD.
Osaka-shi
Osaka
530-8565 (JP)**

(72) Inventors:
• **KAWAMURA, Daichi**
**Mishima-gun, Osaka 618-0021 (JP)**
• **OGATA, Yuudai**
**Mishima-gun, Osaka 618-0021 (JP)**
• **AKAMINE, Takayuki**
**Mishima-gun, Osaka 618-0021 (JP)**
• **TODA, Tomoki**
**Mishima-gun, Osaka 618-0021 (JP)**

(74) Representative: **Ter Meer Steinmeister & Partner
Patentanwälte mbB
Nymphenburger Straße 4
80335 München (DE)**

(56) References cited:
WO-A1-2016/121805    WO-A1-2017/014306
WO-A1-2017/014306    WO-A1-2017/115838
JP-A- H06 510 279    JP-A- 2014 172 840
JP-A- 2017 154 989    US-A1- 2009 142 388
US-A1- 2018 185 273

• **MINGHETTI, P. et al.: "Application of viscometry
and solubility parameters in miconazole patches
development", Int J Pharm, vol. 190, 1999, pages
91-101, XP000925684, ISSN: 0378-5173, DOI:
10.1016/S0378-5173(99)00282-3**

Remarks:
The file contains technical information submitted after
the application was filed and not included in this
specification

EP 3 821 883 B1

## Description

### TECHNICAL FIELD

The present invention relates to an adhesive composition and an adhesive patch containing the adhesive composition.

### BACKGROUND ART

In recent years, adhesive patches have been increasingly developed that are capable of making active ingredients such as drugs transdermally absorbed.

For example, Patent Document 1 below discloses an adhesive patch including an adhesive layer containing Solid-in-Oil type fine particles and an acrylic polymer. The Solid-in-Oil type fine particles are formed from a hydrophilic drug covered with a surfactant.

Patent Document 2 below discloses an adhesive patch including a support, and an adhesive layer holding a drug and provided on the support. The adhesive layer contains a composite of a Solid-in-Oil (S/O) type hydrophilic compound and a surfactant, a thermoplastic elastomer, and an oily ingredient. Patent Document 2 also discloses that the content of the thermoplastic elastomer is 10% by mass or more and less than 40% by The formulation of WO-2017014306 includes a core-shell structure, provided with a core portion containing an active ingredient and a shell portion covering at least part of the surface of the core portion and containing a surfactant, and at least one polymer selected from the group consisting of styrene elastomers having a styrene content of 10-50% by weight, polymers having aminoalkyl groups, and polymers having structural units based on a monomer with an SP value of 8.7-12.

#### Related Art Documents

#### Patent Documents

Patent Document 1: JP 2014-172840 A
Patent Document 2: JP 2017-154989 A

### SUMMARY OF THE INVENTION

### PROBLEMS TO BE SOLVED BY THE INVENTION

When the adhesive patch of Patent Document 1 or 2 is used, however, the transdermal absorbability of the active ingredient is still insufficient. In addition, when an attempt is made to improve the transdermal absorbability of the active ingredient, there are cases where the adhesive properties such as holding power are deteriorated or the adhesive patch is peeled off from the affected area during use. Therefore, it has been difficult to achieve both the transdermal absorbability of the active ingredient and adhesive properties such as holding power.

An object of the present invention is to provide an adhesive composition capable of improving both the transdermal absorbability of the active ingredient and adhesive properties such as holding power, and an adhesive patch containing the adhesive composition.

### MEANS FOR SOLVING THE PROBLEMS

The present inventors have conducted intensive studies, and as a result, found that the following adhesive composition can solve the above-mentioned problems: an adhesive composition that contains a core-shell structure containing an active ingredient, a first elastomer, and a second elastomer different from the first elastomer, and that has a difference between the SP value of the first elastomer and the SP value of the second elastomer within a specific range. Thus, the present inventors have accomplished the present invention.

That is, the adhesive composition according to the present invention is an adhesive composition containing: a core-shell structure including a core portion containing an active ingredient, and a shell portion covering at least part of a surface of the core portion and containing a surfactant; a first elastomer; and a second elastomer different from the first elastomer, the adhesive composition having a difference between the SP value of the first elastomer and the SP value of the second elastomer of 0.1 or more and 3.0 or less.

In a specific aspect of the adhesive composition according to the present invention, the first elastomer and the second elastomer each have an SP value of 8 or more and 11 or less.

In another specific aspect of the adhesive composition according to the present invention, a mixture of the first elastomer and the second elastomer has a haze measured in accordance with JIS K 7361 of 3 or more and 75 or less.

In still another specific aspect of the adhesive composition according to the present invention, the adhesive composition has a weight ratio of the second elastomer to the first elastomer (second elastomer/first elastomer) of 0.1 or more and 10 or less.

In still another specific aspect of the adhesive composition according to the present invention, the first elastomer is an acrylic elastomer.

In still another specific aspect of the adhesive composition according to the present invention, the second elastomer is at least one elastomer selected from the group consisting of an acrylic elastomer, a styrene elastomer, an olefin elastomer, a polyisoprene, a polyisobutylene, a urethane elastomer, and a silicone elastomer.

In still another specific aspect of the adhesive composition according to the present invention, the surfactant includes at least one surfactant selected from the group consisting of a sorbitan fatty acid ester, a glycerol fatty acid ester, a propylene glycol fatty acid ester, and a fatty acid alkanolamide.

In still another specific aspect of the adhesive composition according to the present invention, the glycerol fatty acid ester is at least one ester selected from the group consisting of a monoglycerol fatty acid ester, a diglycerol fatty acid ester, and a triglycerol fatty acid ester.

In still another specific aspect of the adhesive composition according to the present invention, the adhesive composition has a weight ratio of the active ingredient to the surfactant (active ingredient : surfactant) of 1 : 0.1 to 1 : 100.

The adhesive patch according to the present invention includes a support, and an adhesive layer that is laminated on the support and that contains the adhesive composition formed according to the present invention.

## EFFECT OF THE INVENTION

According to the present invention, it is possible to provide an adhesive composition capable of improving both the transdermal absorbability of the active ingredient and adhesive properties such as holding power, and an adhesive patch containing the adhesive composition.

## BRIEF DESCRIPTION OF DRAWINGS

[Fig. 1] Fig. 1 is a schematic cross-sectional view showing an adhesive patch according to one embodiment of the present invention.
[Fig. 2] Fig. 2 is a simplified diagram of a drug skin permeation test cell.

## MODES FOR CARRYING OUT THE INVENTION

Hereinafter, details of the present invention will be described.

[Adhesive composition]

The adhesive composition of the present invention contains a core-shell structure, a first elastomer, and a second elastomer. The core-shell structure includes a core portion containing an active ingredient, and a shell portion containing a surfactant. The shell portion covers at least part of a surface of the core portion. The second elastomer is an elastomer different from the first elastomer. The adhesive composition has a difference between the SP value of the first elastomer and the SP value of the second elastomer of 0.1 or more and 3.0 or less.

Since the difference between the SP value of the first elastomer and the SP value of the second elastomer is within the above-mentioned range, the adhesive composition of the present invention is capable of improving both the transdermal absorbability of the active ingredient and adhesive properties such as holding power.

If the difference in the SP value is less than 0.1, the first elastomer and the second elastomer become highly compatible with each other, and the releasability of the core-shell structure from the adhesive composition may be deteriorated, so that the transdermal absorbability of the active ingredient may be insufficient. On the contrary, if the difference in the SP value exceeds 3.0, the first elastomer and the second elastomer become poorly compatible with each other, and the core-shell structure may be excessively released from the adhesive composition and tends to be unevenly distributed at the interface, so that the adhesive properties such as holding power may be insufficient.

Herein, the "SP value" means a calculated value that is calculated by the Fedors equation: $\delta^2 = \Sigma E / \Sigma V$ (wherein $\delta$ is the SP value, E is the evaporation energy, and V is the molar volume) . The unit of the SP value is $(cal/cm^3)^{0.5}$. The

Fedors method is described in the Journal of the Adhesion Society of Japan, vol. 22, p. 566 (1986).

In the present invention, the difference between the SP value of the first elastomer and the SP value of the second elastomer is preferably 0.5 or more, more preferably 1 or more, and is preferably 2.5 or less, more preferably 2 or less. In this case, both the transdermal absorbability of the active ingredient and adhesive properties such as holding power can be further improved.

Hereinafter, constituent components of the adhesive composition of the present invention will be described in more detail.

(Core-shell structure)

In the present invention, the core-shell structure includes a core portion containing an active ingredient, and a shell portion containing a surfactant. The core portion and the shell portion may be linked together by an intermolecular force or the like to form an aggregate. However, from the viewpoint of further improving the transdermal absorbability of the active ingredient, it is preferable that at least part of the surface of the core portion be covered with the shell portion.

More specifically, it is preferable that 30% or more of the surface of the core portion be covered with the shell portion. More preferably 50% or more, still more preferably 70% or more, still more preferably 85% or more, particularly preferably 95% or more, most preferably 99% or more of the surface of the core portion is covered with the shell portion. Note, however, that the surface of the core portion may be completely covered with the shell portion. Since the core-shell structure has the above-mentioned structure, when, for example, the adhesive patch is applied to the skin, the core-shell structure can release the active ingredient contained in the core portion into the body.

In the present invention, the core portion is preferably solid. When the core portion is solid, the stability in the adhesive layer described later can be further improved. In this case, the core-shell structure can be dispersed in the adhesive layer that is an oil phase to form an adhesive patch having a S/O (Solid-in-Oil) type structure.

As described in the section of "Production method" described later, the core-shell structure in which the core portion is solid (S in the S/O (Solid-in-Oil) type structure) is obtained, for example, by drying a W/O emulsion to remove the solvents (an aqueous solvent and an oil solvent). It is preferable that the step of drying the W/O emulsion substantially completely remove the moisture. Specifically, for example, the water content as measured by the Karl Fischer method is preferably 5% by weight or less, more preferably 2% by weight or less, still more preferably 1% by weight or less, particularly preferably 0.5% by weight or less. Therefore, it is preferable that the core-shell structure be different from the W/O emulsion.

The shape of the core-shell structure is not particularly limited, and the core-shell structure may be, for example, spherical particles. However, the core-shell structure may be particles having a rod-like, cubic, lens-like, micellar, lamellar, hexagonal, bicellular, sponge-like, or sea urchin-like shape, or may have an irregular shape.

The size of the core-shell structure is also not particularly limited. From the viewpoint of further improving the transdermal absorbability of the active ingredient, the core-shell structure may preferably have an average size of 1 nm to 100 $\mu$m.

The average size of the core-shell structure refers to the number average size calculated by a dynamic light scattering method in a state where the core-shell structure is dispersed in a solvent (for example, squalane) using, for example, "Zetasizer Nano S" manufactured by Malvern Panalytical Ltd.

In the present invention, the content of the core-shell structure is not particularly limited, but is preferably 10 parts by weight or more, more preferably 15 parts by weight or more, still more preferably 20 parts by weight or more, and is preferably 60 parts by weight or less, more preferably 50 parts by weight or less, still more preferably 40 parts by weight or less based on 100 parts by weight of the entire adhesive composition. When the content of the core-shell structure is equal to or more than the above-mentioned lower limit, transdermal absorbability can be further improved. When the content of the core-shell structure is equal to or less than the above-mentioned upper limit, adhesive properties such as holding power can be further improved.

Core portion;

The core portion contains at least an active ingredient.

Specific examples of the active ingredient include, but are not particularly limited to, anti-dementia drugs, antiepileptic drugs, antidepressants, antiparkinsonian drugs, anti-allergic drugs, anticancer drugs, diabetes drugs, hypotensive drugs, respiratory disease drugs, anti-ED drugs, dermatological drugs, and local anesthetics. The active ingredients may be used alone, or in combination of two or more types.

More specific examples of the active ingredient include memantine, donepezil, diphenhydramine, vardenafil, octreotide, rivastigmine, galantamine, nitroglycerin, lidocaine, fentanyl, male hormones, female hormones, nicotine, clomipramine, nalfurafine, metoprolol, fesoterodine, tandospirone, beraprost sodium, taltirelin, lurasidone, nefazodone, rifaximin, benidipine, doxazosin, nicardipine, formoterol, lomerizine, amlodipine, teriparatide, bucladesine, cromoglicic acid, lixisenatide, exenatide, liraglutide, lanreotide, glucagon, oxytocin, calcitonin, elcatonin, glatiramer, risedronic acid, diclofenac, ascorbic acid, and pharmaceutically acceptable salts of these compounds.

The pharmaceutically acceptable salt is not particularly limited, and either of an acidic salt and a basic salt can be employed. Examples of the acidic salt include inorganic acid salts such as hydrochlorides, hydrobromides, sulfates, nitrates, and phosphates, and organic acid salts such as acetates, propionates, tartrates, fumarates, maleates, malates, citrates, methanesulfonates, benzenesulfonates, and p-toluenesulfonates. Examples of the basic salt include alkali metal salts such as sodium salts and potassium salts, and alkaline earth metal salts such as calcium salts and magnesium salts. Specific examples of the salt of active ingredient include memantine hydrochloride, donepezil hydrochloride, rivastigmine tartrate, galantamine hydrobromide, clomipramine hydrochloride, diphenhydramine hydrochloride, nalfurafine hydrochloride, metoprolol tartrate, fesoterodine fumarate, vardenafil hydrochloride hydrate, nalfurafine hydrochloride, tandospirone citrate, beraprost sodium, lurasidone hydrochloride, nefazodone hydrochloride, benidipine hydrochloride, doxazosin mesylate, nicardipine hydrochloride, formoterol fumarate, lomerizine hydrochloride, and amlodipine besylate.

The active ingredient blended in cosmetics is not particularly limited as long as the active ingredient is required to have skin permeation properties. Examples of such active ingredient include vitamin ingredients such as vitamin C and vitamin E, moisturizing ingredients such as hyaluronic acid, ceramide, and collagen, whitening ingredients such as tranexamic acid and arbutin, hair growth ingredients such as minoxidil, beauty ingredients such as FGFs (fibroblast growth factors) and EGFs (epidermal growth factors), and salts and derivatives of these compounds.

The active ingredient is preferably hydrophilic. When the active ingredient is a hydrophilic drug, a drug that is required to have systemic action or local action is usually used.

The active ingredient is preferably a drug that can be easily absorbed transdermally. The active ingredient is not particularly limited, but is preferably a compound having an octanol/water partition coefficient of -2 to 6. In this case, the skin permeation properties of the active ingredient are further improved. From the viewpoint of further improving the skin permeation properties of the active ingredient, the octanol/water partition coefficient is preferably -1 or more, more preferably 0 or more. The octanol/water partition coefficient of the active ingredient is preferably 4 or less, more preferably 1 or less. When the octanol/water partition coefficient of the active ingredient is equal to or less than the above-mentioned upper limit, the skin permeation properties of the active ingredient are further improved.

In the present invention, the octanol/water partition coefficient is determined from the active ingredient concentrations in the octanol and aqueous phases, through adding the active ingredient in a flask containing octanol and an aqueous buffer solution of pH 7, and then shaking the flask. Specifically, the octanol/water partition coefficient can be obtained by calculating the equation: octanol/water partition coefficient = $Log_{10}$ (concentration in octanol phase/concentration in aqueous phase).

The amount of the active ingredient contained in the core-shell structure depends on the type of the active ingredient, but is, for example, preferably 1% by weight to 70% by weight, more preferably 5% by weight to 70% by weight in terms of the raw material weight. The raw material weight is a value based on the total weight of all the raw materials contained in the core-shell structure.

The core portion may contain two or more types of active ingredients as necessary.

The molecular weight of the active ingredient is not particularly limited. The molecular weight of the active ingredient is preferably 250 g/mol or more, more preferably 300 g/mol or more, and is preferably 7,500 g/mol or less, more preferably 6,500 g/mol or less, still more preferably 1,500 g/mol or less.

Shell portion;

The shell portion contains at least a surfactant. The HLB value of the surfactant is is 4 or more and 14 or less, more preferably 5 or more and 12 or less. When the shell portion contains a plurality of surfactants, it is preferable that the weighted average of the HLB values of the surfactants be within the above-mentioned range.

The HLB (an abbreviation of Hydrophile Lypophile Balance) value is an index that indicates whether an emulsifier is hydrophilic or lipophilic, and assumes a value of 0 to 20. The smaller the HLB value is, the higher the lipophilicity is.

The HLB value is calculated from the following Griffin equation.

$$\text{HLB value} = 20 \times \{(\text{molecular weight of hydrophilic portion})/(\text{total molecular weight})\}$$

The weighted average of the HLB values can be calculated using, for example, the following calculation formula.

As for surfactants having HLB values of A, B, and C, and weights of x, y, and z, respectively, the formula for calculating the weighted average of the HLB values is (xA + yB + zC)/(x + y + z).

It is preferable that the surfactant have at least one of a saturated hydrocarbon group such as an alkyl group, and an unsaturated hydrocarbon group such as an alkenyl group and an alkynyl group. The hydrocarbon group of the surfactant include a saturated hydrocarbon group having 7 to 15 carbon atoms or an unsaturated hydrocarbon group having 7 to 17 carbon atoms.

When the surfactant contains a plurality of hydrocarbon groups, the hydrocarbon group contained at the largest proportion in the surfactant is defined as the hydrocarbon group of the surfactant in the present invention.

In particular, when the surfactant contains a plurality of hydrocarbon groups different in the number of carbon atoms, the number of carbon atoms of a hydrocarbon group contained at the largest proportion in the surfactant is defined as the number of carbon atoms of the hydrocarbon group of the surfactant.

For example, specifically, when the surfactant is a coconut oil fatty acid ester, the surfactant contains a saturated hydrocarbon group having 11 carbon atoms at the largest proportion. Therefore, the hydrocarbon group of the coconut oil fatty acid ester is the saturated hydrocarbon group, and the number of carbon atoms of the hydrocarbon group is 11.

The number of carbon atoms of the saturated hydrocarbon group is preferably 7 or more and 15 or less, more preferably 7 or more and 11 or less. When the number of carbon atoms of the saturated hydrocarbon group is equal to or more than the above-mentioned lower limit, the covering properties of the shell portion to cover the surface of the core portion are further improved. When the number of carbon atoms of the saturated hydrocarbon group is equal to or less than the above-mentioned upper limit, the releasability of the core-shell structure to release the active ingredient in the body are further improved.

The number of carbon atoms of the unsaturated hydrocarbon group is preferably 7 or more and 17 or less, more preferably 7 or more and 13 or less, still more preferably 7 or more and 11 or less. When the number of carbon atoms of the unsaturated hydrocarbon group is equal to or more than the above-mentioned lower limit, the covering properties of the shell portion to cover the surface of the core portion are further improved. When the number of carbon atoms of the unsaturated hydrocarbon group is equal to or less than the above-mentioned upper limit, the releasability of the core-shell structure to release the active ingredient in the body are further improved.

The molecular weight of the hydrophilic portion of the surfactant is preferably 100 g/mol or more and 350 g/mol or less, more preferably 100 g/mol or more and 300 g/mol or less, still more preferably 100 g/mol or more and 200 g/mol or less. When the molecular weight of the hydrophilic portion of the surfactant is equal to or more than the above-mentioned lower limit, the covering properties of the shell portion to cover the core portion are further improved. When the molecular weight of the hydrophilic portion of the surfactant is equal to or less than the above-mentioned upper limit, the releasability of the core-shell structure to release the active ingredient in the body are further improved.

The "hydrophilic portion" of the surfactant refers to the portion other than the hydrocarbon group of the constituent fatty acid in the whole surfactant molecule. For example, in the case of sorbitan monooleate, the whole surfactant molecule has a molecular weight of 428.6 g/mol, and the hydrocarbon group of monooleic acid, which is the constituent fatty acid of the surfactant, has a molecular weight of 237.4 g/mol. Therefore, the molecular weight of the hydrophilic portion of the surfactant is obtained by subtracting the molecular weight of the hydrocarbon group of the constituent fatty acid from the molecular weight of the whole surfactant molecule, and is calculated as 191.2 g/mol.

The surfactant includes at least one surfactant selected from the group consisting of a sorbitan fatty acid ester, a glycerol fatty acid ester, a propylene glycol fatty acid ester, and a fatty acid alkanolamide. Above all, it is preferable that the surfactant include at least one surfactant selected from the group consisting of a sorbitan fatty acid ester, a glycerol fatty acid ester, and a propylene glycol fatty acid ester from the viewpoint of achieving both the transdermal absorbability and low skin irritation at a higher level.

The sorbitan fatty acid ester is not particularly limited, and examples thereof include an ester of sorbitan and a fatty acid.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, and erucic acid. These fatty acids may be fatty acids derived from natural fats and oils such as beef tallow, lard, coconut oil, palm oil, palm kernel oil, olive oil, rapeseed oil, rice bran oil, soybean oil, and castor oil.

Preferable specific examples of the sorbitan fatty acid ester include, from the viewpoint of further improving the transdermal absorbability of the active ingredient, sorbitan monostearate (NIKKOL SS-10MV manufactured by NIPPON SURFACTANT INDUSTRIES CO., LTD.), sorbitan trioleate (NIKKOL SO-30V manufactured by NIPPON SURFACTANT INDUSTRIES CO., LTD.), sorbitan sesquioleate (NIKKOL SO-15MV manufactured by NIPPON SURFACTANT INDUSTRIES CO., LTD.), sorbitan monooleate (NIKKOL SO-10V manufactured by NIPPON SURFACTANT INDUSTRIES CO., LTD.), sorbitan monolaurate (NIKKOL SL-10 manufactured by NIPPON SURFACTANT INDUSTRIES CO., LTD.), coconut oil fatty acid sorbitan (EMALEX SPC-10 manufactured by Nihon Emulsion Co., Ltd.), and sorbitan laurate (RIKEMAL L-250A manufactured by RIKEN VITAMIN CO.,

LTD.) .

The glycerol fatty acid ester in the present invention is not particularly limited, and examples thereof include an ester of glycerol and a fatty acid.

The glycerol may be polyglycerol. The degree of polymerization n of polyglycerol is not particularly limited, but is preferably 5 or less, more preferably 4 or less, still more preferably 3 or less. Above all, it is preferable that the glycerol be monoglycerol, diglycerol, or triglycerol. Specifically, the glycerol fatty acid ester is preferably a monoglycerol fatty acid ester, a diglycerol fatty acid ester, or a triglycerol fatty acid ester. In this case, transdermal absorption of the active ingredient can be further improved.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, and erucic acid. These fatty acids may be fatty acids derived from natural fats and oils such as beef tallow, lard, coconut oil, palm oil, palm kernel oil, olive oil, rapeseed oil, rice bran oil, soybean oil, and castor oil.

Preferable specific examples of the glycerol fatty acid ester include, from the viewpoint of further improving the transdermal absorbability of the active ingredient, diglyceryl monostearate (NIKKOL DGMS manufactured by NIPPON SURFACTANT INDUSTRIES CO., LTD.), glyceryl monostearate (NIKKOL MGS-BMV manufactured by NIPPON SURFACTANT INDUSTRIES CO., LTD.), glyceryl monostearate (NIKKOL MGS-AMV manufactured by NIPPON SURFACTANT INDUSTRIES CO., LTD.), glyceryl monostearate (NIKKOL MGS-DEXV manufactured by NIPPON SURFACTANT INDUSTRIES CO., LTD.), glyceryl monostearate (NIKKOL MGS-ASEV manufactured by NIPPON SURFACTANT INDUSTRIES CO., LTD.), glyceryl monostearate (NIKKOL MGS-BSEV manufactured by NIPPON SURFACTANT INDUSTRIES CO., LTD.), glyceryl myristate (MGM manufactured by NIPPON SURFACTANT INDUSTRIES CO., LTD.), glyceryl tri(caprylate/caprate) (NIKKOL TRIESTER F-810 manufactured by NIPPON SURFACTANT INDUSTRIES CO., LTD.), glyceryl monooleate (NIKKOL MGO manufactured by NIPPON SURFACTANT INDUSTRIES CO., LTD.), glyceryl monooleate (Capmul GMO-50 manufactured by ABITEC Corporation), glyceryl olivate (NIKKOL MGOL-70 manufactured by NIPPON SURFACTANT INDUSTRIES CO., LTD.), diglyceryl monooleate (NIKKOL DGMO-CV manufactured by NIPPON SURFACTANT INDUSTRIES CO., LTD.), diglyceryl monooleate (NIKKOL DGMO-90V manufactured by NIPPON SURFACTANT INDUSTRIES CO., LTD.), glyceryl monocaprylate (Sunsoft No. 700P-2-C manufactured by Taiyo Kagaku Co., Ltd.), glyceryl monocaprylate (Capmul 808G manufactured by ABITEC Corporation), glyceryl monocaprylate (Capmul MCM C8 manufactured by ABITEC Corporation), glyceryl monocaprate (Sunsoft No. 760-C manufactured by Taiyo Kagaku Co., Ltd.), glyceryl caprate (Capmul MCM C10 manufactured by ABITEC Corporation), glyceryl caprylate/caprate (Capmul MCM manufactured by ABITEC Corporation), glyceryl caprylate/caprate (Capmul 471 manufactured by ABITEC Corporation), capric acid mono- and di-glycerides (Sunsoft No. 707-C manufactured by Taiyo Kagaku Co., Ltd.), capric acid diglyceride (Sunfat GDC-S manufactured by Taiyo Kagaku Co., Ltd.), glyceryl monolaurate (Sunsoft No. 750-C manufactured by Taiyo Kagaku Co., Ltd.), and glyceryl monoundecylenate (NIKKOL MGU manufactured by NIPPON SURFACTANT INDUSTRIES CO., LTD.).

More preferable examples of the glycerol fatty acid ester include glyceryl monooleate (NIKKOL MGO manufactured by NIPPON SURFACTANT INDUSTRIES CO., LTD.), glyceryl monooleate (Capmul GMO-50 manufactured by ABITEC Corporation), glyceryl olivate (NIKKOL MGOL-70 manufactured by NIPPON SURFACTANT INDUSTRIES CO., LTD.), diglyceryl monooleate (NIKKOL DGMO-CV manufactured by NIPPON SURFACTANT INDUSTRIES CO., LTD.), diglyceryl monooleate (NIKKOL DGMO-90V manufactured by NIPPON SURFACTANT INDUSTRIES CO., LTD.), glyceryl monocaprylate (Sunsoft No. 700P-2-C manufactured by Taiyo Kagaku Co., Ltd.), glyceryl monocaprylate (Capmul 808G manufactured by ABITEC Corporation), glyceryl monocaprylate (Capmul MCM C8 manufactured by ABITEC Corporation), glyceryl monocaprate (Sunsoft No. 760-C manufactured by Taiyo Kagaku Co., Ltd.), glyceryl caprate (Capmul MCM C10 manufactured by ABITEC Corporation), glyceryl caprylate/caprate (Capmul MCM manufactured by ABITEC Corporation), glyceryl caprylate/caprate (Capmul 471 manufactured by ABITEC Corporation), capric acid mono- and di-glycerides (Sunsoft No. 707-C manufactured by Taiyo Kagaku Co., Ltd.), capric acid diglyceride (Sunfat GDC-S manufactured by Taiyo Kagaku Co., Ltd.), glyceryl monolaurate (Sunsoft No. 750-C manufactured by Taiyo Kagaku Co., Ltd.), and glyceryl monoundecylenate (NIKKOL MGU manufactured by NIPPON SURFACTANT INDUSTRIES CO., LTD.).

The propylene glycol fatty acid ester is not particularly limited, and examples thereof include an ester of propylene glycol and a fatty acid.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, and erucic acid. These fatty acids may be fatty acids derived from natural fats and oils such as beef tallow, lard, coconut oil, palm oil, palm kernel oil, olive oil, rapeseed oil, rice bran oil, soybean oil, and castor oil.

Preferable specific examples of the propylene glycol fatty acid ester include, from the viewpoint of further improving the transdermal absorbability of the active ingredient, propylene glycol monostearate (RIKEMAL PS-100 manufactured by RIKEN VITAMIN CO., LTD.), propylene glycol monostearate (NIKKOL PMS-1CV manufactured by NIPPON SURFACTANT INDUSTRIES CO., LTD.), propylene glycol diisostearate (EMALEX PG-di-IS manufactured by Nihon Emulsion Co., Ltd.), propylene glycol distearate (EMALEX PG-di-S manufactured by Nihon Emulsion Co., Ltd.), preferably propylene glycol monolaurate (RIKEMAL PL-100 manufactured by RIKEN VITAMIN CO., LTD.), propylene glycol monooleate (RIKEMAL PO-100 manufactured by RIKEN VITAMIN CO., LTD.), propylene glycol dioleate (EMALEX PG-di-O manufactured by Nihon Emulsion Co., Ltd.), propylene glycol dicaprylate (NIKKOL SEFSOL-228 manufactured by NIPPON SURFACTANT INDUSTRIES CO., LTD.), and propylene glycol dilaurate (EMALEX PG-M-L manufactured by Nihon Emulsion Co., Ltd.) .

The "fatty acid alkanolamide" refers to a compound that has a structure in which an R-CO group and two -$CH_2CH_2OH$ groups are bonded together with N being the center, and that is represented by the chemical formula R-$CON(CH_2CH_2OH)_2$.

Specific examples of the fatty acid alkanolamide include oleic acid diethanolamide, lauric acid diethanolamide, lauric acid monoisopropanolamide, stearic acid diethanolamide, stearic acid monoethanolamide, stearic acid monoisopropanolamide, lauramide/myristamide DEA, palmitic acid monoethanolamide, coconut oil fatty acid diethanolamide, coconut oil fatty acid monoisopropanolamide, coconut oil fatty acid N-methylethanolamide, coconut oil fatty acid monoethanolamide, and palm kernel oil fatty acid diethanolamide. From the viewpoint of further improving the skin permeation properties, the fatty acid alkanolamide is preferably a diethanolamide such as oleic acid diethanolamide, lauric acid diethanolamide, and coconut oil fatty acid diethanolamide.

In the present invention, the surfactant may further include a surfactant other than the sorbitan fatty acid ester, the glycerol fatty acid ester, the propylene glycol fatty acid ester, and the fatty acid alkanolamide, and such other surfactant can be appropriately selected according to the intended use. For example, the surfactant can be widely selected from those usable as pharmaceuticals and cosmetics. Further, two or more types of surfactants may be used in combination.

The surfactant other than the sorbitan fatty acid ester, the glycerol fatty acid ester, the propylene glycol fatty acid ester, and the fatty acid alkanolamide may be any of a nonionic surfactant, an anionic surfactant, a cationic surfactant, and an amphoteric surfactant.

The nonionic surfactant is not particularly limited, and examples thereof include fatty acid esters, fatty alcohol ethoxylates, polyoxyethylene alkylphenyl ethers, alkyl glycosides, polyoxyethylene castor oil, and hydrogenated castor oil.

The fatty acid ester is not particularly limited, and examples thereof include esters of at least one of glycerol, polyglycerol, polyoxyethylene glycerol, polyoxyethylene, sorbitan, propylene glycol, polyoxyethylene sorbitol and the like, with caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, erucic acid and the like. These fatty acid esters may be esters with fatty acids derived from natural fats and oils such as beef tallow, lard, coconut oil, palm oil, palm kernel oil, olive oil, rapeseed oil, rice bran oil, soybean oil, and castor oil.

Examples of the anionic surfactant include alkylsulfate ester salts, polyoxyethylene alkyl ether sulfate ester salts, alkylbenzene sulfonate salts, fatty acid salts, and phosphate ester salts.

Examples of the cationic surfactant include alkyl trimethyl ammonium salts, dialkyl dimethyl ammonium salts, alkyl dimethyl benzyl ammonium salts, and amine salts.

Examples of the amphoteric surfactant include alkylamino fatty acid salts, alkyl betaines, and alkylamine oxides.

The surfactant other than the sorbitan fatty acid ester, the glycerol fatty acid ester, the propylene glycol fatty acid ester, and the fatty acid alkanolamide is particularly preferably a sucrose fatty acid ester, a polyoxyethylene glycerol fatty acid ester, a polyoxyethylene sorbitol fatty acid ester, polyoxyethylene castor oil, or hydrogenated castor oil.

The surfactant other than the sorbitan fatty acid ester, the glycerol fatty acid ester, the propylene glycol fatty acid ester, and the fatty acid alkanolamide may have a hydrocarbon chain such as an alkyl chain, an alkenyl chain, or an alkynyl chain.

The content of the surfactant can be appropriately decided within a range in which the effects of the present invention are exhibited, but the weight ratio of the active ingredient to the surfactant (active ingredient : surfactant) is preferably adjusted to 1 : 0.5 to 1 : 100, more preferably to 1 : 5 to 1 : 100. In this case, the transdermal absorbability of the active ingredient can be further improved. From the viewpoint of further improving the transdermal absorbability of the active ingredient, the weight ratio of the active ingredient to the surfactant (active ingredient : surfactant) is more preferably adjusted to 1 : 0.5 to 1 : 50, particularly preferably to 1 : 0.5 to 1 : 30. From the viewpoint of further improving the absorbability of the active ingredient, the weight ratio of the active ingredient to the surfactant (active ingredient : surfactant) is more preferably adjusted to 1 : 5 to 1 : 50, particularly preferably to 1 : 5 to 1 : 30.

Further, in the present invention, the weight ratio of the active ingredient to the surfactant (active ingredient : surfactant) may be 1 : 0.5 to 1 : 2. Usually, in a tape preparation, the higher the content of the active ingredient is, the more the dispersibility of the active ingredient in the tape preparation tends to deteriorate. However, when a surfactant having the above-mentioned HLB value or a saturated hydrocarbon group or an unsaturated hydrocarbon group is used, even if the content of the active ingredient is high, the dispersibility of the active ingredient in the tape preparation can be further improved.

Other additive components;

The core-shell structure may further contain, in addition to the active ingredient and the surfactant, at least one other component. Such other component is not particularly limited, and examples thereof include stabilizers, penetration enhancers, skin irritation reducing agents, antiseptics, and analgesics.

The stabilizer has a function of stabilizing the particle structure. The stabilizer also has a role of preventing unintended early collapse of the particle structure and further improving the sustained release effect of the active ingredient.

The stabilizer is not particularly limited, and examples thereof include polysaccharides, proteins, and hydrophilic polymer materials. The core-shell structure may contain one type or two or more types of stabilizers. The content of the stabilizer can be appropriately decided according to the type thereof. For example, the stabilizer can be blended so that the weight ratio of the active ingredient to the stabilizer (active ingredient : stabilizer) may be 1 : 0.1 to 1 : 10.

The penetration enhancer is not particularly limited, and examples thereof include higher alcohols, N-acyl sarcosine and salts thereof, higher monocarboxylic acids, higher monocarboxylic acid esters, aromatic monoterpene fatty acid esters, divalent carboxylic acids having 2 to 10 carbon atoms and salts thereof, polyoxyethylene alkyl ether phosphate esters and salts thereof, lactic acid, lactic acid esters, and citric acid. The core-shell structure may contain one type or two or more types of penetration enhancers. The content of the penetration enhancer can be appropriately decided according to the type thereof. For example, the penetration enhancer can be blended so that the weight ratio of the active ingredient to the penetration enhancer (active ingredient : penetration enhancer) may be 1 : 0.01 to 1 : 50.

The skin irritation reducing agent is not particularly limited, and examples thereof include hydroquinone glycosides, pantethine, tranexamic acid, lecithin, titanium oxide, aluminum hydroxide, sodium nitrite, sodium hydrogen nitrite, soybean lecithin, methionine, glycyrrhetinic acid, BHT, BHA, vitamin E and derivatives thereof, vitamin C and derivatives thereof, benzotriazole, propyl gallate, and mercaptobenzimidazole. The core-shell structure may contain one type or two or more types of skin irritation reducing agents. The content rate of the skin irritation reducing agent can be appropriately decided according to the type thereof. The skin irritation reducing agent can be blended so that the content rate of the skin irritation reducing agent may be 0.1% by weight to 50% by weight based on the entire core-shell structure.

The antiseptic is not particularly limited, and examples thereof include methyl parahydroxybenzoate, propyl parahydroxybenzoate, phenoxyethanol, and thymol. The content rate of the antiseptic in the core portion can be appropriately decided according to the type thereof. The antiseptic can be blended so that the content rate of the antiseptic may be 0.01% by weight to 10% by weight based on the entire core-shell structure. The core-shell structure may contain one type or two or more types of antiseptics.

The analgesic is not particularly limited, and examples thereof include local anesthetics such as procaine, tetracaine, lidocaine, dibucaine, and prilocaine, and salts thereof. The core-shell structure may contain one type or two or more types of analgesics. The content rate of the analgesic in the core-shell structure can be appropriately decided according to the type thereof. The analgesic can be blended so that the content rate of the analgesic may be 0.1% by weight to 30% by weight based on the entire core-shell structure.

Production method;

The method for producing the core-shell structure is not particularly limited. For example, the core-shell structure can be produced by a method including a step of drying a W/O emulsion containing an active ingredient in an aqueous phase.

The W/O emulsion is not particularly limited as long as it is a so-called water-in-oil emulsion, specifically, an emulsion containing droplets of an aqueous solvent dispersed in an oil solvent.

The W/O emulsion containing an active ingredient in an aqueous phase can be obtained, for example, by mixing an aqueous solvent such as water or a buffered aqueous solution, which contains an active ingredient, with an oil solvent such as cyclohexane, hexane, or toluene, which contains a surfactant. The aqueous solvent containing an active ingredient may contain, in addition to the active ingredient, additive components such as stabilizers, absorption enhancers, and irritation reducing agents as necessary. Further, the oil solvent containing a surfactant may contain, in addition to the surfactant, additive components such as irritation reducing agents, analgesics, absorption enhancers, and stabilizers as necessary. The method of mixing is not particularly limited as long as the method can form a W/O emulsion, and may be, for example, stirring with a homogenizer or the like.

Conditions for the stirring with a homogenizer may be, for example, about 5,000 rpm to 50,000 rpm, preferably about 10,000 rpm to 30,000 rpm.

The weight ratio of the active ingredient to the surfactant (active ingredient : surfactant) in the W/O emulsion is preferably within the range of 1 : 0.5 to 1 : 100, more preferably within the range of 1 : 5 to 1 : 100. The weight ratio (active ingredient : surfactant) is still more preferably within the range of 1 : 0.5 to 1 : 50, particularly preferably within the range of 1 : 5 to 1 : 50. Further, the weight ratio (active ingredient : surfactant) is more preferably within the range of 1 : 0.5 to 1 : 30, particularly preferably within the range of 1 : 5 to 1 : 30. The weight ratio of the active ingredient to the surfactant (active ingredient : surfactant) may be 1 : 0.5 to 1 : 2.

The method for drying the W/O emulsion containing an active ingredient in an aqueous phase is not particularly limited as long as the method can remove the solvents (an aqueous solvent and an oil solvent) in the emulsion. Examples of the method for drying the W/O emulsion include freeze drying and drying under reduced pressure, preferably freeze drying.

From the viewpoint of further reducing the number average particle size of the obtained core-shell structure, it is preferable that the method further include a step of heat-treating the W/O emulsion or a dried product of the W/O emulsion. The heat treatment temperature is, for example, 30°C to 60°C, preferably 35°C to 50°C, more preferably 35°C to 45°C.

The heat treatment time is appropriately adjusted according to the heat treatment temperature, and is, for example, 1 day to 30 days, preferably 2 days to 15 days, more preferably 3 to 7 days.

Further, as another method for further reducing the number average particle size of the obtained core-shell structure, there can be mentioned a method of dispersing the W/O emulsion or a dried product of the W/O emulsion in a solvent or the like as necessary, and then filtering the resulting dispersion using a filter or the like, and a method of performing centrifugal separation. The filter pore size in the case of filter filtration is, for example, 1 um or less, preferably 0.2 um or less, more preferably 0.1 um or less.

(First elastomer)

The first elastomer is not particularly limited, and examples thereof include acrylic elastomers, styrene elastomers, olefin elastomers, polyisoprenes, polyisobutylenes, urethane elastomers, and silicone elastomers. These may be used alone, or in combination of two or more types. Above all, it is preferable to use an acrylic elastomer as the first elastomer.

Examples of the acrylic elastomer include a polymer of a (meth)acrylic monomer. The "meth(acrylic)" refers to methacrylic or acrylic.

Specific examples of the (meth)acrylic monomer include acrylic acid, dodecyl acrylate, acrylic acid octyl esters, 2-ethylhexyl acrylate, hydroxyethyl acrylate, acrylic acid esters, octyl acrylate, methyl acrylate, butyl acrylate, n-octadecyl acrylate, ethyl acrylate, alkyl acrylate, acrylic acid alkyl esters, octylamide acrylate, 2-methoxyethyl acrylate, hydroxypropyl acrylate, methoxyethyl acrylate, methacrylic acid, ethyl methacrylate, glycidyl methacrylate, octyl methacrylate, 2-ethylhexyl methacrylate, dodecyl methacrylate, acetoacetoxyethyl methacrylate, methyl methacrylate, 2-methoxyethyl methacrylate, methacrylic acid alkyl esters, alkyl methacrylate, and n-octadecyl methacrylate. These may be homopolymers or copolymers of two or more types of the above-mentioned (meth)acrylic monomers.

The acrylic elastomer may be a copolymer of a (meth)acrylic monomer and a different monomer. Examples of the different monomer include vinyl acetate, vinyl pyrrolidone, acrylamide monomers, styrene monomers, vinyl ether monomers, diacetone acrylamide monomers, and acrylamide monomers.

Further, the method employed for polymerizing the methacrylic monomer may be a conventionally known method, and examples thereof include a method using a polymerization initiator. Examples of the polymerization initiator include azobis polymerization initiators such as 2,2'-azobisisobutyronitrile (AIBN), 1,1'-azobis(cyclohexane-1-carbonitrile), and 2,2'-azobis-(2,4'-dimethylvaleronitrile), and organic peroxides such as benzoyl peroxide (BPO), lauroyl peroxide (LPO), and di-tert-butyl peroxide. The polymerization initiator is preferably lauroyl peroxide, benzoyl peroxide or the like. The polymerization initiators may be used alone, or in combination of two or more types.

Examples of the acrylic elastomer include an acrylic acid/acrylic acid octyl ester copolymer, a 2-ethylhexyl acrylate/vinyl acetate/hydroxyethyl acrylate/glycidyl methacrylate copolymer, an acrylic acid ester/vinyl acetate copolymer, a 2-ethylhexyl acrylate/methyl acrylate/acrylic acid/glycidyl methacrylate copolymer, a 2-ethylhexyl acrylate/diacetone acrylamide/acetoacetoxyethyl methacrylate/methyl methacrylate copolymer, a 2-ethylhexyl acrylate/vinyl pyrrolidone copolymer, a 2-ethylhexyl acrylate/2-ethylhexyl methacrylate/dodecyl methacrylate copolymer, a methyl acrylate/2-ethylhexyl acrylate copolymer, an acrylic acid/2-ethylhexyl acrylate copolymer, an acrylic acid/butyl acrylate copolymer, an acrylic acid/2-ethylhexyl acrylate/vinyl acetate copolymer, an acrylic acid/2-ethylhexyl acrylate/hydroxyethyl acrylate copolymer, an acrylic acid/hydroxyethyl acrylate/vinyl pyrrolidone copolymer, an acrylic acid/2-ethylhexyl acrylate/hydroxyethyl acrylate/vinyl pyrrolidone copolymer, an acrylic acid/acrylic acid amide/ethyl acrylate copolymer, an acrylic acid/2-ethylhexyl acrylate/styrene copolymer, an acrylic acid amide/styrene copolymer, an acrylic acid alkyl ester/methacrylic acid alkyl ester/diacetone acrylamide/methacrylic acid copolymer, an alkyl acrylate/vinyl acetate copolymer, an alkyl acrylate/styrene copolymer, an octylamide acrylate/acrylic acid ester copolymer, a hydroxyethyl acrylate/butyl acrylate/methoxyethyl acrylate copolymer, a hydroxyethyl acrylate/methoxyethyl acrylate copolymer, a butyl acrylate/acrylonitrile/styrene copolymer, and an acrylic acid/alkyl methacrylate copolymer. It is also possible to add a crosslinking agent as necessary. Examples of the crosslinking agent include amino compounds, phenol compounds, epoxy compounds, isocyanate compounds, organic peroxides, metal alcoholates, and metal chelates.

Examples of the styrene elastomer include styreneisoprene-styrene block copolymers (SIS), styrene-butadienestyrene block copolymers (SBS), styrene-ethylene butylene-styrene block copolymers (SEBS), styrene-ethylenepropylene-styrene block copolymers (SEPS), and styrene-isobutylene-styrene block copolymers (SIBS).

Examples of the olefin elastomer include crystalline olefin-ethylene butene-crystalline olefin (CEBC) copolymers and styrene-ethylene butene-crystalline olefin (SEBC) copolymers.

The SP value of the first elastomer is preferably 8 or more, more preferably 8.5 or more, still more preferably 9 or more, particularly preferably 9.5 or more, and is preferably 12 or less, more preferably 11.5 or less, still more preferably 11 or less, particularly preferably 10.5 or less. When the SP value of the first elastomer is within the above-mentioned range, both the transdermal absorbability of the active ingredient and adhesive properties such as holding power can be further improved.

The first elastomer is preferably an acrylic elastomer or a styrene elastomer, and is more preferably an acrylic elastomer, from the viewpoint of good dispersibility of the core-shell structure.

(Second elastomer)

The second elastomer is an elastomer different from the first elastomer. Further, the second elastomer is an elastomer having a smaller SP value than that of the first elastomer.

The second elastomer is not particularly limited, and examples thereof include acrylic elastomers, styrene elastomers, olefin elastomers, polyisoprenes, polyisobutylenes, urethane elastomers, and silicone elastomers. These may be used alone, or in combination of two or more types. Above all, it is preferable to use an acrylic elastomer, a styrene elastomer, or a polyisoprene as the second elastomer. These elastomers may be, for example, those listed in the section of the first elastomer.

Note that the second elastomer may be of the same type as the first elastomer. Specifically, when the first elastomer is an acrylic elastomer, the second elastomer may be a different acrylic elastomer.

The SP value of the second elastomer is preferably 6.5 or more, more preferably 7 or more, still more preferably 7.5 or more, particularly preferably 8 or more, and is preferably 11 or less, more preferably 10.5 or less, still more preferably 10 or less, particularly preferably 9.5 or less. When the SP value of the second elastomer is within the above-mentioned range, both the transdermal absorbability of the active ingredient and adhesive properties such as holding power can be further improved.

The second elastomer is preferably an acrylic elastomer or a styrene elastomer, and is more preferably an acrylic elastomer, from the viewpoint of good dispersibility of the core-shell structure.

In the present invention, the sum of the contents of the first elastomer and the second elastomer is not particularly limited, but is preferably 20 parts by weight or more, more preferably 30 parts by weight or more, and is preferably 80 parts by weight or less, more preferably 70 parts by weight or less, still more preferably 60 parts by weight or less based on 100 parts by weight of the entire adhesive composition. When the sum of the contents of the first elastomer and the second elastomer is within the above-mentioned range, both the transdermal absorbability of the active ingredient and adhesive properties such as holding power can be further improved.

The combination of the first elastomer and the second elastomer is preferably a combination in which one is an acrylic elastomer and the other is a styrene elastomer, or both the elastomers are acrylic elastomers, and is more preferably a combination in which both the elastomers are acrylic elastomers. In this case, the dispersibility of the core-shell structure can be more effectively improved.

The weight ratio of the second elastomer to the first elastomer (second elastomer/first elastomer) is preferably 0.1 or more, more preferably 0.2 or more, and is preferably 10 or less, more preferably 5 or less. When the weight ratio (second elastomer/first elastomer) is within the above-mentioned range, both the transdermal absorbability of the active ingredient and adhesive properties such as holding power can be further improved.

The haze of the mixture of the first elastomer and the second elastomer measured in accordance with JIS K 7361 is preferably 3 or more, more preferably 3.5 or more, still more preferably 4 or more, and is preferably 75 or less, more preferably 50 or less, still more preferably 30 or less. When the haze of the mixture of the first elastomer and the second elastomer is within the above-mentioned range, both the transdermal absorbability of the active ingredient and adhesive properties such as holding power can be further improved.

The haze can be measured, for example, under the following conditions.

In accordance with JIS K 7361, a 100-pm-thick mixture of the first elastomer and the second elastomer is placed on a 1-mm-thick glass substrate to produce a measurement sample, and the measurement sample is placed in a haze meter ("HM-150" manufactured by MURAKAMI COLOR RESEARCH LABORATORY) and subjected to the measurement of the haze value (%) in an environment at room temperature of 25°C and 40% humidity.

The 100-pm-thick mixture of the first elastomer and the second elastomer can be produced, for example, as follows. Note that the following production method is an exemplary method in the examples. The mixture may be produced by other methods, and the production method is not limited.

The method for producing the 100-pm-thick mixture of the first elastomer and the second elastomer is not particularly limited. For example, in the examples, the mixture was produced as follows.

To a mixture of the first elastomer and the second elastomer, toluene is added so that the resulting mixture may have a solid content concentration of 45% by weight, and then the components are mixed until a uniform mixture is obtained to prepare a mixed solution of the first elastomer and the second elastomer. Then, to a release-treated surface of a release sheet, which is obtained by applying silicone to one surface of a release base made of a 38-μm-thick polyethylene terephthalate film to subject the release base to release treatment, the mixed solution of the first elastomer and the second elastomer is applied. Then, the mixed solution is dried at 90°C for 20 minutes to provide the 100-pm-thick mixture of the first elastomer and the second elastomer on the release-treated surface of the release sheet.

In the measurement of the haze, the weight ratio of the second elastomer to the first elastomer (second elastomer/first elastomer) in the mixture of the first elastomer and the second elastomer is equal to the weight ratio of the second elastomer to the first elastomer (second elastomer/first elastomer) in the adhesive composition.

When the adhesive composition of the present invention contains three or more types of elastomers, two types of elastomers each having a larger weight ratio in the adhesive composition are selected, and the elastomer having a larger SP value is defined as the first elastomer and the elastomer having a smaller SP value is defined as the second elastomer.

(Tackifying resin)

The adhesive composition of the present invention may further contain a tackifying resin. The tackifying resin is not particularly limited, and examples thereof include alicyclic saturated hydrocarbon resins, terpene resins, terpene phenol resins, hydrogenated terpene resins, hydrogenated terpene phenol resins, and rosin derivatives. The tackifying resin is preferably an alicyclic saturated hydrocarbon resin, a rosin derivative or the like. The tackifying resins may be used alone, or in combination of two or more types.

The content of the tackifying resin is not particularly limited, and may be, for example, 1 part by weight or more and 40 parts by weight or less based on 100 parts by weight of the entire adhesive composition. When the content of the tackifying resin is within the above-mentioned range, both the transdermal absorbability of the active ingredient and adhesive properties such as holding power can be further improved.

(Other additives)

The adhesive composition of the present invention may contain other additive components and the like according to the purpose of use and the like. Examples of other additive components include elastomers different from the first elastomer and the second elastomer, resins, plasticizers, gelling agents, excipients, coloring agents, lubricants, binders, emulsifiers, thickeners, wetting agents, stabilizers, preservatives, solvents, dissolution aids, suspending agents, buffers, pH adjusters, antioxidants, penetration enhancers, irritation mitigating agents, antiseptics, chelating agents, and dispersants.

The plasticizer used is preferably an oily solution. However, the plasticizer may be an aqueous solution.

Examples of the oily solution as the plasticizer include vegetable oils, animal oils, neutral lipids, synthetic fats and oils, sterol derivatives, waxes, hydrocarbons, alcohol carboxylic acid esters, oxyacid esters, polyhydric alcohol fatty acid esters, silicones, higher alcohols, higher fatty acids, and fluorinated oils. Examples of the aqueous solution include water and (polyhydric) alcohols. The plasticizer is preferably an oily solution such as a hydrocarbon, an alcohol carboxylic acid ester, a polyhydric alcohol fatty acid ester, and an oxyacid ester. The plasticizers may be used alone, or in combination of two or more types.

The plasticizer may be gelated. Here, the "gelation" means that molecules in a liquid containing small molecules or macromolecules or a liquid formed of small molecules or macromolecules partially crosslink with each other to form a three-dimensional network structure. The plasticizer may be gelated by physical or chemical crosslinking.

The gelling agent is not particularly limited as long as the plasticizer can be gelated by the gelling agent, and examples thereof include an ester of one or more fatty acids and one polysaccharide. The fatty acid is preferably a fatty acid having 5 to 26 carbon atoms, more preferably a fatty acid having 6 to 18 carbon atoms. The polysaccharide is preferably dextrin, inulin, sucrose or the like. The gelling agents may be used alone, or in combination of two or more types.

The content of other additives is not particularly limited, and may be, for example, 0.1 parts by weight or more and 20 parts by weight or less based on 100 parts by weight of the entire adhesive composition.

[Adhesive patch]

The adhesive patch of the present invention is not particularly limited, and examples thereof include tape preparations such as plasters and emplastra (such as those of reservoir type and matrix type), cataplasms, patches, and microneedles. Above all, it is preferable that the adhesive patch be a tape preparation.

The adhesive patch of the present invention can be used after being appropriately cut into, for example, an elliptical, circular, square, or rectangular shape according to the intended use.

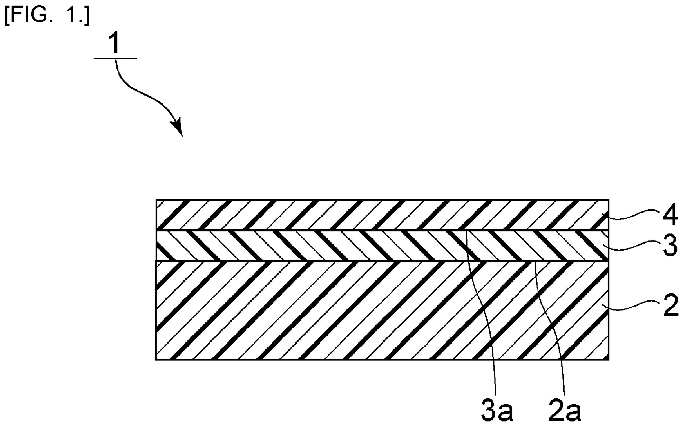

Hereinafter, an example of the adhesive patch of the present invention will be described with reference to Fig. 1.

Fig. 1 is a schematic cross-sectional view showing an adhesive patch according to one embodiment of the present invention.

An adhesive patch 1 is a tape preparation. As shown in Fig. 1, the adhesive patch 1 includes a support 2 and an adhesive layer 3. The adhesive layer 3 is laminated on a surface 2a of the support 2. On a surface 3a of the adhesive layer 3, a liner 4 is laminated.

The adhesive layer 3 may be laminated only on the surface 2a on one side of the support 2 as in the present embodiment, or may be laminated on both surfaces of the support 2. The adhesive layer 3 of the adhesive patch 1 is formed from the adhesive composition of the present invention, and contains a core-shell structure. However, in an adhesive patch of a reservoir type or the like, the core-shell structure does not have to be contained in the adhesive layer 3, and may be contained, for example, in a reservoir phase.

The support 2 is not particularly limited as long as it supports the adhesive layer 3, and examples thereof include a resin film, a fiber, and a nonwoven fabric. Examples of the resin film include films of polyesters and polyolefins. The resin film is preferably a polyester film. Examples of the polyester include polyethylene terephthalate and polybutylene phthalate, and polyethylene terephthalate is preferable.

The liner 4 is not particularly limited as long as it protects the adhesive layer 3 until the adhesive patch 1 is applied to the skin and is coated with, for example, silicone so that it can be easily peeled off. Examples of the liner 4 include polyethylene terephthalate or polypropylene coated with silicone. The liner 4 does not have to be provided. To form the adhesive layer 3, an adhesive layer solution described later may be applied either to the support 2 or to the liner 4.

Since the adhesive patch 1 has the adhesive layer 3 formed from the adhesive composition of the present invention, both the transdermal absorbability of the active ingredient and adhesive properties such as holding power can be improved.

Hereinafter, a specific example of the method for producing the adhesive patch of the present invention will be described.

(Method for producing adhesive patch)

The method for producing the adhesive patch of the present invention is not particularly limited, and the adhesive patch can be produced by a solution coating method. In the solution coating method, first, an adhesive base phase solution (adhesive layer solution) is prepared.

As for a method for preparing the adhesive base phase solution, for example, first, the above-mentioned adhesive composition of the present invention is mixed in a solvent. The adhesive base phase solution can thus be prepared.

The method of mixing the components is not particularly limited, and a known method can be used. For example, the components can be mixed by stirring with a magnetic stirrer under conditions of 500 rpm for 1 hour.

Examples of the solvent include cyclohexane, hexane, methylcyclohexane, toluene, heptane, ethyl acetate, n-butyl acetate, isobutyl acetate, isopropyl acetate, methyl acetate, propyl acetate, tetrahydrofuran, acetone, pentane, methyl isobutyl ketone, and methyl ethyl ketone.

The solid content concentration of the adhesive base phase solution is preferably 10 to 80% by weight, more preferably 20 to 60% by weight.

Then, the adhesive base phase solution is uniformly applied to the liner using a coating machine such as a knife coater, a comma coater, or a reverse coater, and dried. The solvent is thus removed to complete the adhesive base phase layer (adhesive layer), and the support is laminated on the adhesive base phase layer, whereby the adhesive patch can be obtained. Note that an aging step may be provided. The aging may be performed, for example, at 20 to 80°C for 1 to 7 days. The aging may be performed after the adhesive base phase solution is applied and dried, or after the support is laminated on the adhesive base phase layer. The liner and the support used may be those described above, respectively.

In the following, the present invention will be clarified with reference to specific examples of the present invention and comparative examples. Note that the present invention is not limited to the following examples.

(Example 1)

As an active ingredient, 2.0 g of donepezil hydrochloride (manufactured by Tokyo Chemical Industry Co., Ltd., molecular weight: 416) was dissolved in 38 g of pure water. To the resulting solution, a solution obtained by dissolving 2.0 g of glyceryl monolaurate (trade name "Sunsoft No. 750-C" manufactured by Taiyo Kagaku Co., Ltd., HLB value:

8.7) as a surfactant in 78 g of cyclohexane was added, and the resulting mixture was stirred with a homogenizer (25,000 rpm). Then, the mixture was freeze-dried for 2 days to produce particles as a core-shell structure having a structure including a core portion containing the active ingredient and a shell portion containing the surfactant. To 40 parts by weight of the obtained particles, 20 parts by weight in terms of solid content of an acrylic elastomer as a first elastomer, 30 parts by weight in terms of solid content of an acrylic elastomer as a second elastomer, and 10 parts by weight of a tackifier were blended, and toluene was added so that the resulting mixture should have a solid content concentration of 45% by weight. Then, the components were mixed until a uniform mixture was obtained to prepare an adhesive layer solution. The acrylic elastomer used as the first elastomer was trade name "MAS683" (a 2-ethylhexyl acrylate/vinyl pyrrolidone copolymer, SP value of the polymer: 10.4) manufactured by CosMED Pharmaceutical Co., Ltd. The acrylic elastomer used as the second elastomer was trade name "MAS811B" (a 2-ethylhexyl acrylate/2-ethylhexyl methacrylate/dodecyl methacrylate copolymer, SP value of the polymer: 9.1) manufactured by CosMED Pharmaceutical Co., Ltd. The tackifier used was trade name "Pine Crystal KE-311" (a rosin ester) manufactured by ARAKAWA CHEMICAL INDUSTRIES, LTD.

Then, a release sheet was prepared, which was obtained by applying silicone to one surface of a release base made of a 38-um-thick polyethylene terephthalate film to subject the release base to release treatment. The prepared adhesive layer solution was applied to the release-treated surface of the release sheet and dried at 90°C for 20 minutes to produce a laminate including the release sheet and a 100-pm adhesive layer formed on the release-treated surface of the release sheet. Then, a support made of a 38-um-thick polyethylene terephthalate film was prepared. The support and the laminate were stacked so that one surface of the support might face the adhesive layer of the laminate, and the adhesive layer of the laminate was transferred to the support to laminate and integrate the laminate and the support, whereby an adhesive patch was produced.

(Example 2)

An adhesive patch was produced in the same manner as in Example 1 except that a styrene-isoprene-styrene block copolymer (SIS, trade name "Quintac 3520", Q 3520 manufactured by Zeon Corporation, styrene content: 15%, amount of diblock: 78%, SP value: 8.1 to 8.5) was blended as the second elastomer instead of the acrylic elastomer.

(Example 3)

An adhesive patch was produced in the same manner as in Example 1 except that to 60 parts by weight of particles obtained in the same manner as in Example 1, 13.3 parts by weight in terms of solid content of an acrylic elastomer as a first elastomer, 20 parts by weight in terms of solid content of an acrylic elastomer as a second elastomer, and 6.7 parts by weight of a tackifier were blended. The acrylic elastomer used as the first elastomer was trade name "MAS683" (a 2-ethylhexyl acrylate/vinyl pyrrolidone copolymer, SP value of the polymer: 10.4) manufactured by CosMED Pharmaceutical Co., Ltd. The acrylic elastomer used as the second elastomer was trade name "MAS811B" (a 2-ethylhexyl acrylate/2-ethylhexyl methacrylate/dodecyl methacrylate copolymer, SP value of the polymer: 9.1) manufactured by CosMED Pharmaceutical Co., Ltd. The tackifier used was trade name "Pine Crystal KE-311" (a rosin ester) manufactured by ARAKAWA CHEMICAL INDUSTRIES, LTD.

(Example 4)

An adhesive patch was produced in the same manner as in Example 1 except that 1.0 g of glyceryl monolaurate (trade name "Sunsoft No. 750-C" manufactured by Taiyo Kagaku Co., Ltd., HLB value: 8.7) and 1.0 g of glyceryl monocaprylate (trade name "Sunsoft No. 700P-2-C" manufactured by Taiyo Kagaku Co., Ltd., HLB value: 10.9) were used as the surfactant instead of 2.0 g of glyceryl monolaurate (trade name "Sunsoft No. 750-C" manufactured by Taiyo Kagaku Co., Ltd., HLB value: 8.7).

(Example 5)

An adhesive patch was produced in the same manner as in Example 1 except that to 40 parts by weight of particles obtained in the same manner as in Example 1, 45.5 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 4.5 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Example 6)

An adhesive patch was produced in the same manner as in Example 1 except that to 40 parts by weight of particles obtained in the same manner as in Example 1, 8.3 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 41.7 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Example 7)

An adhesive patch was produced in the same manner as in Example 1 except that to 40 parts by weight of particles obtained in the same manner as in Example 1, 4.5 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 45.5 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Example 8)

An adhesive patch was produced in the same manner as in Example 1 except that to 40 parts by weight of particles obtained in the same manner as in Example 1, 45.5 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 4.5 parts by weight in terms of solid content of a styrene-isoprene-styrene block copolymer (SIS, trade name "Quintac 3520", Q 3520 manufactured by Zeon Corporation, styrene content: 15%, amount of diblock: 78%, SP value: 8.1 to 8.5) as a second elastomer, and 10 parts by weight of a tackifier (trade name "Pine Crystal KE-311" manufactured by ARAKAWA CHEMICAL INDUSTRIES, LTD., a rosin ester) were blended.

(Example 9)

An adhesive patch was produced in the same manner as in Example 1 except that to 40 parts by weight of particles obtained in the same manner as in Example 1, 8.3 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 41.7 parts by weight in terms of solid content of a styrene-isoprene-styrene block copolymer (SIS, trade name "Quintac 3520", Q 3520 manufactured by Zeon Corporation, styrene content: 15%, amount of diblock: 78%, SP value: 8.1 to 8.5) as a second elastomer, and 10 parts by weight of a tackifier (trade name "Pine Crystal KE-311" manufactured by ARAKAWA CHEMICAL INDUSTRIES, LTD., a rosin ester) were blended.

(Example 10)

An adhesive patch was produced in the same manner as in Example 1 except that to 40 parts by weight of particles obtained in the same manner as in Example 1, 4.5 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 45.5 parts by weight in terms of solid content of a styrene-isoprene-styrene block copolymer (SIS, trade name "Quintac 3520", Q 3520 manufactured by Zeon Corporation, styrene content: 15%, amount of diblock: 78%, SP value: 8.1 to 8.5) as a second elastomer, and 10 parts by weight of a tackifier (trade name "Pine Crystal KE-311" manufactured by ARAKAWA CHEMICAL INDUSTRIES, LTD., a rosin ester) were blended.

(Example 11)

An adhesive patch was produced in the same manner as in Example 1 except that to 30 parts by weight of particles obtained in the same manner as in Example 1, 24 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 36 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Example 12)

An adhesive patch was produced in the same manner as in Example 1 except that to 70 parts by weight of particles obtained in the same manner as in Example 1, 8 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, 12 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Example 13)

An adhesive patch was produced in the same manner as in Example 1 except that to 40 parts by weight of particles obtained in the same manner as in Example 1, 20 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a first elastomer, 30 parts by weight in terms of solid content of a styrene-isoprene-styrene block copolymer (SIS, trade name "Quintac 3520", Q 3520 manufactured by Zeon Corporation, styrene content: 15%, amount of diblock: 78%, SP value: 8.1 to 8.5) as a second elastomer, and 10 parts by weight of a tackifier (trade name "Pine Crystal KE-311" manufactured by ARAKAWA CHEMICAL INDUSTRIES, LTD., a rosin ester) were blended.

(Comparative Example 1)

An adhesive patch was produced in the same manner as in Example 1 except that 50 parts by weight in terms of solid content of an acrylic elastomer (trade name "MAS683" manufactured by CosMED Pharmaceutical Co., Ltd., a 2-ethylhexyl acrylate/vinyl pyrrolidone copolymer, SP value of the polymer: 10.4) as a first elastomer, and 10 parts by weight of a tackifier (trade name "Pine Crystal KE-311" manufactured by ARAKAWA CHEMICAL INDUSTRIES, LTD., a rosin ester) were blended, and no second elastomer was used.

(Comparative Example 2)

An adhesive patch was produced in the same manner as in Example 1 except that 50 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended, and no first elastomer was used.

(Comparative Example 3)

An adhesive patch was produced in the same manner as in Example 2 except that 50 parts by weight of the styrene-isoprene-styrene block copolymer as a second elastomer, and 10 parts by weight of the tackifier were blended, and no first elastomer was used. The styrene-isoprene-styrene block copolymer (SIS) used was trade name "Quintac 3520" (Q 3520, styrene content: 15%, amount of diblock: 78%, SP value: 8.1 to 8.5) manufactured by Zeon Corporation. The tackifier used was trade name "Pine Crystal KE-311" manufactured by ARAKAWA CHEMICAL INDUSTRIES, LTD., a rosin ester.

(Comparative Example 4)

An adhesive patch was produced in the same manner as in Example 1 except that to 40 parts by weight of particles obtained in the same manner as in Example 1, 20 parts by weight in terms of solid content of polyvinyl alcohol (trade name "Poly(vinyl Alcohol) 3,500" manufactured by FUJIFILM Wako Pure Chemical Corporation, SP value of the polymer: 12.6) as a first elastomer, 30 parts by weight in terms of solid content of an acrylic elastomer (trade name "MAS811B" manufactured by CosMED Pharmaceutical Co., Ltd., a 2-ethylhexyl acrylate/2-ethylhexyl methacrylate/dodecyl methacrylate copolymer, SP value of the polymer: 9.1) as a second elastomer, and 10 parts by weight of a tackifier (trade name "Pine Crystal KE-311" manufactured by ARAKAWA CHEMICAL INDUSTRIES, LTD., a rosin ester) were blended.

(Evaluation)

Haze;

Mixtures that were blends of the first elastomer and the second elastomer used in each of the examples and comparative examples as shown in Table 1 were produced. Then, the haze of the produced mixtures was measured in accordance with JIS K 7361.

Specifically, the mixture adjusted to a thickness of 100 um was placed on a 1-mm-thick glass substrate to produce a measurement sample, and the measurement sample was placed in a haze meter ("HM-150" manufactured by MURAKAMI COLOR RESEARCH LABORATORY) and subjected to the measurement of the haze value (%) in an environment at room temperature of 25°C and 40% humidity.

[Table 1]

| | First elastomer (parts by weight) | Second elastomer (parts by weight) | Second elastomer/ first elastomer (mass ratio) |
|---|---|---|---|
| Example 1 | 20 | 30 | 1.5 |
| Example 2 | 20 | 30 | 1.5 |
| Example 3 | 13.3 | 20 | 1.5 |
| Example 4 | 20 | 30 | 1.5 |
| Example 5 | 45.5 | 4.5 | 0.1 |
| Example 6 | 8.3 | 41.7 | 5.0 |
| Example 7 | 4.5 | 45.5 | 10.0 |
| Example 8 | 45.5 | 4.5 | 0.1 |
| Example 9 | 8.3 | 41.7 | 5.0 |
| Example 10 | 4.5 | 45.5 | 10.0 |
| Example 11 | 24.0 | 36.0 | 1.5 |
| Example 12 | 8 | 12 | 1.5 |
| Example 13 | 20 | 30 | 1.5 |
| Comparative Example 1 | 50 | 0 | 0.0 |
| Comparative Example 2 | 0 | 50 | - |
| Comparative Example 3 | 0 | 50 | - |
| Comparative Example 4 | 20 | 30 | 1.5 |

Hairless rat skin permeation test;

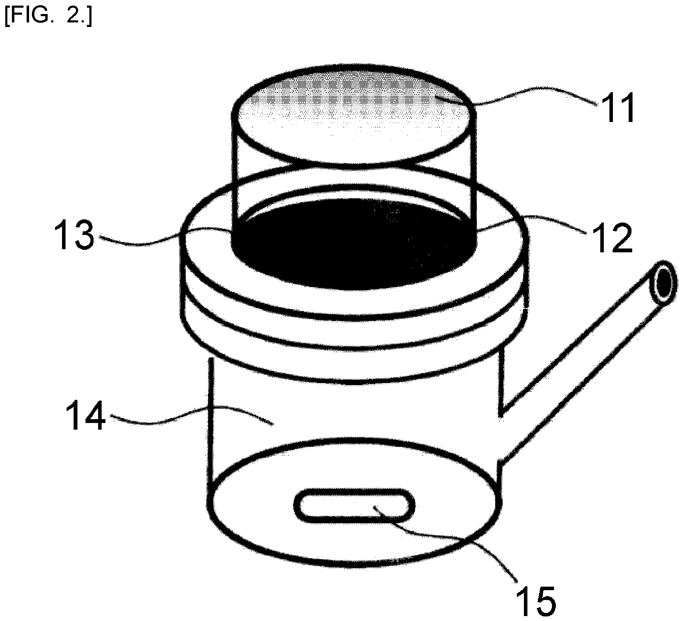

Hairless rat skin (removed from HWY/Slc, 8-week-old rat available from Japan SLC, Inc.) was placed on a drug skin permeation test cell (Fig. 2). On top of the device, 1.33 $cm^2$ of the adhesive patch produced in each of the examples and comparative examples was applied. A liquid containing $5 \times 10^{-4}$ M of $NaH_2PO_4$, $2 \times 10^{-4}$ M of $Na_2HPO_4$, $1.5 \times 10^{-4}$ M of NaCl, and 10 ppm of gentamicin sulfate (G1658 manufactured by Wako Pure Chemical Industries, Ltd.) in distilled water was adjusted to pH 7.2 with NaOH to prepare a buffer solution, and the buffer solution was added to a receptor layer positioned at the bottom of the device. The device was placed in a thermostat kept at 32°C from the start of the test. After a predetermined time from the start of the test, 1 ml of the liquid in the vessel was collected from the receptor layer at the bottom, and immediately after that, 1 ml of a liquid having the same composition was replenished. To each of the collected receptor liquid samples, methanol was added to extract eluted lipids and the like, followed by centrifugation. After the centrifugation, the concentration of the active ingredient in the supernatant was quantified by high performance liquid chromatography (HPLC). Based on the quantified amount of the active ingredient, a 24-hour cumulative skin permeation amount (permeation amount after 24 hours) was calculated.

Peel force;

In accordance with JIS Z 0237:2009, the adhesive patch having a width of 24 mm and a length of 100 mm was attached to a SUS plate to produce a test piece, and the test piece was peeled off in the direction of 90°. The strength at the time of peeling was defined as the peel force. The peel force was determined by measurement with a tensile tester. The tensile tester used was product number "SVZ-50NB-1R1" manufactured by IMADA-SS Corporation. The peeling rate was 300 mm/min.

Holding power (holding time);

In accordance with JIS Z 0237:2009, the adhesive patch having a width of 12 mm and a length of 12 mm was attached to a SUS plate. The elapsed time (holding time) from when a 1.0 kg weight was attached to an end of the adhesive patch until the adhesive patch completely peeled off from the SUS plate was measured. A holding power tester used was product number "BE-502" manufactured by TESTER SANGYO CO., LTD.
The results are shown in Tables 2 and 3 below.
In Tables 2 and 3, whether or not both the skin permeation properties and adhesiveness were satisfied was evaluated according to the following evaluation criteria.

<Evaluation criteria>

Excellent ... The adhesive patch satisfies all of a permeation amount after 24 hours of 100 $\mu$g/cm$^2$ or more, a peel force of 50 mN/mm or more, and a holding time of 50 seconds or more.
Good ... The adhesive patch satisfies all of a permeation amount after 24 hours of 51 $\mu$g/cm$^2$ or more, a peel force of 31 mN/mm or more, and a holding time of 10 seconds or more.
Poor ... The adhesive patch satisfies only one or none of a permeation amount after 24 hours of 51 $\mu$g/cm$^2$ or more, a peel force of 31 mN/mm or more, and a holding time of 10 seconds or more.

[Table 2]

| | Core-shell structure | | | First elastomer | | Second elastomer | | Second elastomer/ first elastomer (mass ratio) | Difference in SP value | Haze of film without particles | Permeation amount after 24 hours ($\mu$g/cm$^2$) | Peel force (mN/mm) | Holding time (sec) | Combination of permeation properties and adhesiveness |
|---|---|---|---|---|---|---|---|---|---|---|---|---|---|---|
| | Drug | Surfactant | Particle concentration | Name | SP value | Name | SP value | | | | | | | |
| Example 1 | Donepezil | Glyceryl monolaurate | 40% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 1.5 | 1.3 | 5.19 | 440 | 210 | >300 | Excellent |
| Example 2 | Donepezil | Glyceryl monolaurate | 40% by weight | 2-Ethylhexyl acrylate/vinyl pyrrolidone copolymer | 10.4 | Styrene-isoprene-styrene block copolymer | 8.1-8.5 | 1.5 | 1.9-2.3 | 70.75 | 313 | 50 | >300 | Excellent |
| Example 3 | Donepezil | Glyceryl monolaurate | 60% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 1.5 | 1.3 | 5.19 | 150 | 160 | >300 | Excellent |
| Example 4 | Donepezil | Glyceryl monocaprylate and glyceryl monolaurate | 40% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 1.5 | 1.3 | 5.19 | 446 | 180 | >300 | Excellent |

EP 3 821 883 B1

19

| | Core-shell structure | | | First elastomer | | Second elastomer | | Second elastomer/ first elastomer (mass ratio) | Difference in SP value | Haze of film without particles | Permeation amount after 24 hours ($\mu$g/cm$^2$) | Peel force (mN/mm) | Holding time (sec) | Combination of permeation properties and adhesiveness |
|---|---|---|---|---|---|---|---|---|---|---|---|---|---|---|
| | Drug | Surfactant | Particle concentration | Name | SP value | Name | SP value | | | | | | | |
| Example 5 | Donepezil | Glyceryl monolaurate | 40% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 0.1 | 1.3 | 3.27 | 130 | 182 | 192 | Excellent |
| Example 6 | Donepezil | Glyceryl monolaurate | 40% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 5 | 1.3 | 6.31 | 259 | 92 | 162 | Excellent |
| Example 7 | Donepezil | Glyceryl monolaurate | 40% by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 10 | 1.3 | 5.22 | 96 | 42 | 68 | Good |
| Example 8 | Donepezil | Glyceryl monolaurate | 40% by weight | 2-Ethylhexyl acrylate/vinyl pyrrolidone copolymer | 10.4 | Styrene-isoprene-styrene block copolymer | 8.1-8.5 | 0.1 | 1.9-2.3 | 71.83 | 113 | 186 | 170 | Excellent |

(continued)

| | | Core-shell structure | | First elastomer | | Second elastomer | | Second elastomer/ first elastomer (mass ratio) | Difference in SP value | Haze of film without particles | Permeation amount after 24 hours ($\mu$g/cm$^2$) | Peel force (mN/mm) | Holding time (sec) | Combination of permeation properties and adhesiveness |
|---|---|---|---|---|---|---|---|---|---|---|---|---|---|---|
| | Drug | Surfactant | Particle concentration | Name | SP value | Name | SP value | | | | | | | |
| Example 9 | Donepezil | Glyceryl monolaurate | 40% by weight | 2-Ethylhexyl acrylate/vinyl pyrrolidone copolymer | 10.4 | Styrene-isoprene-styrene block copolymer | 8.1-8.5 | 5 | 1.9-2.3 | 58.73 | 236 | 150 | 151 | Excellent |
| Example 10 | Donepezil | Glyceryl monolaurate | 40% by weight | 2-Ethylhexyl acrylate/vinyl pyrrolidone copolymer | 10.4 | Styrene-isoprene-styrene block copolymer | 8.1-8.5 | 10 | 1.9-2.3 | 44.98 | 419 | 40 | 72 | Good |
| Example 11 | Donepezil | Glyceryl monolaurate | 30 by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 1.5 | 1.3 | 5.19 | 57 | 132 | >300 | Good |
| Example 12 | Donepezil | Glyceryl monolaurate | 70 by weight | 2-Ethylhexyl acrylate/vinyl pyrrolidone copolymer | 10.4 | 2-Ethylhexyl acrylate/2-ethylhexyl methacrylate/dodecyl methacrylate copolymer | 9.1 | 1.5 | 1.3 | 5.19 | 540 | 35 | 30 | Good |

(continued)

| | Core-shell structure | | | First elastomer | | Second elastomer | | Second elastomer/ first elastomer (mass ratio) | Difference in SP value | Haze of film without particles | Permeation amount after 24 hours ($\mu$g/cm$^2$) | Peel force (mN/mm) | Holding time (sec) | Combination of permeation properties and adhesiveness |
|---|---|---|---|---|---|---|---|---|---|---|---|---|---|---|
| | Drug | Surfactant | Particle concentration | Name | SP value | Name | SP value | | | | | | | |
| Example 13 | Donepezil | Glyceryl monolaurate | 40 by weight | 2-Ethylhexyl acrylate/2-ethylhexyl methacrylate/dodecyl methacrylate copolymer | 9.1 | Styrene-isoprene-styrene block copolymer | 8.1-8.5 | 1.5 | 0.6-1.0 | 4.89 | 54 | 36 | 98 | Good |

[Table 3]

| | Core-shell structure | | | First elastomer | | Second elastomer | | Second elastomer/ first elastomer (mass ratio) | Difference in SP value | Haze of film without particles | Permeation amount after 24 hours ($\mu$g/cm$^2$) | Peel force (mN/mm) | Holding time (sec) | Combination of permeation properties and adhesiveness |
|---|---|---|---|---|---|---|---|---|---|---|---|---|---|---|
| | Drug | Surfactant | Particle concentration | Name | SP value | Name | SP value | | | | | | | |
| Comparative Example 1 | Donepezil | Glyceryl monolaurate | 40% by weight | 2-Ethylhexyl acrylate/vinyl pyrrolidone copolymer | 10.4 | - | - | 1.5 | 0 | 0.62 | 50 | 30 | >300 | Poor |
| Comparative Example 2 | Donepezil | Glyceryl monolaurate | 40% by weight | - | - | 2-Ethylhexyl acrylate/2-ethylhexyl methacrylate/dodecyl methacrylate copolymer | 9.1 | 1.5 | 0 | 1.05 | 88 | 0 | 0 | Poor |
| Comparative Example 3 | Donepezil | Glyceryl monolaurate | 40% by weight | - | - | Styrene-isoprene-styrene block copolymer | 8.1-8.5 | 1.5 | 0 | 2.93 | 28 | 0 | 0 | Poor |
| Comparative Example 4 | Donepezil | Glyceryl monolaurate | 40% by weight | Polyvinyl alcohol | 12,6 | 2-Ethylhexyl acrylate/2-ethylhexyl methacrylate/dodecyl methacrylate copolymer | 9.1 | 1.5 | 3.5 | Evaluation impossible because no uniform film was obtained | | | | |

EXPLANATION OF SYMBOLS

1: Adhesive patch
2: Support
2a, 3a: Surface
3: Adhesive layer
4: Liner
11: Parafilm
12: Skin
13: Adhesive patch
14: Receptor liquid (pH = 7.2 phosphate buffer solution)
15: Stirrer

**Claims**

1. An adhesive composition comprising:

   a core-shell structure including a core portion containing an active ingredient, and a shell portion covering at least part of a surface of the core portion and containing a surfactant;
   a first elastomer; and
   a second elastomer different from the first elastomer,
   the HLB value of the surfactant being 4 or more and 14 or less,
   the surfactant including at least one surfactant selected from the group consisting of a sorbitan fatty acid ester, a glycerol fatty acid ester, a propylene glycol fatty acid ester, and a fatty acid alkanolamide,
   a hydrocarbon group of the surfactant including a saturated hydrocarbon group having 7 to 15 carbon atoms or an unsaturated hydrocarbon group having 7 to 17 carbon atoms,
   the adhesive composition having a weight ratio of the active ingredient to the surfactant (active ingredient : surfactant) may be 1 : 0.5 to 1 . 2, and
   the adhesive composition having a difference between an SP value of the first elastomer and an SP value of the second elastomer of 0.1 or more and 3.0 or less,
   wherein the SP value is determined according to the Fedors method as outlined in the description.

2. The adhesive composition according to claim 1, wherein the first elastomer and the second elastomer each have an SP value of 8 or more and 11 or less,
   wherein the SP value is determined according to the Fedors method as outlined in the description.

3. The adhesive composition according to claim 1 or 2, wherein a mixture of the first elastomer and the second elastomer has a haze measured in accordance with JIS K 7361 of 3 or more and 75 or less.

4. The adhesive composition according to any one of claims 1 to 3, having a weight ratio of the second elastomer to the first elastomer (second elastomer/first elastomer) of 0.1 or more and 10 or less.

5. The adhesive composition according to any one of claims 1 to 4, wherein the first elastomer is an acrylic elastomer.

6. The adhesive composition according to claim 5, wherein the second elastomer is a different acrylic elastomer.

7. The adhesive composition according to any one of claims 1 to 6, wherein the surfactant includes at least one surfactant selected from the group consisting of a sorbitan fatty acid ester, a glycerol fatty acid ester, and a propylene glycol fatty acid ester.

8. The adhesive composition according to any one of claims 1 to 7, wherein the glycerol fatty acid ester is at least one ester selected from the group consisting of a monoglycerol fatty acid ester, a diglycerol fatty acid ester, and a triglycerol fatty acid ester.

9. An adhesive patch comprising:

a support; and
an adhesive layer that is laminated on the support and that contains the adhesive composition according to any one of claims 1 to 8.

**Patentansprüche**

1. Klebstoffzusammensetzung, umfassend:

   eine Kern-Schale-Struktur mit einem Kernteil, der einen Wirkstoff enthält, und einem Schalenteil, der mindestens einen Teil einer Oberfläche des Kernteils bedeckt und ein Tensid enthält,
   ein erstes Elastomer, und
   ein zweites Elastomer, das sich von dem ersten Elastomer unterscheidet,
   wobei der HLB-Wert des Tensids 4 oder mehr und 14 oder weniger beträgt,
   wobei das Tensid mindestens ein Tensid, ausgewählt aus der Gruppe bestehend aus einem Sorbitanfettsäureester, einem Glycerinfettsäureester, einem Propylenglykolfettsäureester und einem Fettsäurealkanolamid, enthält,
   wobei eine Kohlenwasserstoffgruppe des Tensids eine gesättigte Kohlenwasserstoffgruppe mit 7 bis 15 Kohlenstoffatomen oder eine ungesättigte Kohlenwasserstoffgruppe mit 7 bis 17 Kohlenstoffatomen enthält,
   die Klebstoffzusammensetzung ein Gewichtsverhältnis des Wirkstoffs zu dem Tensid (Wirkstoff : Tensid) von 1 : 0,5 bis 1 : 2 aufweist, und
   die Klebstoffzusammensetzung eine Differenz zwischen einem SP-Wert des ersten Elastomers und einem SP-Wert des zweiten Elastomers von 0,1 oder mehr und 3,0 oder weniger aufweist,
   wobei der SP-Wert gemäß der Fedors-Methode, wie in der Beschreibung dargelegt, bestimmt wird.

2. Klebstoffzusammensetzung nach Anspruch 1, wobei das erste Elastomer und das zweite Elastomer jeweils einen SP-Wert von 8 oder mehr und 11 oder weniger aufweisen,
   wobei der SP-Wert gemäß der Fedors-Methode, wie in der Beschreibung dargelegt, bestimmt wird.

3. Klebstoffzusammensetzung nach Anspruch 1 oder 2, wobei ein Gemisch aus dem ersten Elastomer und dem zweiten Elastomer eine nach JIS K 7361 gemessene Trübung von 3 oder mehr und 75 oder weniger aufweist.

4. Klebstoffzusammensetzung nach einem der Ansprüche 1 bis 3, mit einem Gewichtsverhältnis des zweiten Elastomers zu dem ersten Elastomer (zweites Elastomer/erstes Elastomer) von 0,1 oder mehr und 10 oder weniger.

5. Klebstoffzusammensetzung nach einem der Ansprüche 1 bis 4, wobei das erste Elastomer ein Acrylelastomer ist.

6. Klebstoffzusammensetzung nach Anspruch 5, wobei das zweite Elastomer ein anderes Acrylelastomer ist.

7. Klebstoffzusammensetzung nach einem der Ansprüche 1 bis 6, wobei das Tensid mindestens ein Tensid, ausgewählt aus der Gruppe bestehend aus einem Sorbitanfettsäureester, einem Glycerinfettsäureester und einem Propylenglykolfettsäureester, umfasst.

8. Klebstoffzusammensetzung nach einem der Ansprüche 1 bis 7, wobei der Glycerinfettsäureester mindestens ein Ester, ausgewählt aus der Gruppe bestehend aus einem Monoglycerinfettsäureester, einem Diglycerinfettsäureester und einem Triglycerinfettsäureester, ist.

9. Klebepflaster, umfassend:

   einen Träger; und
   eine Klebstoffschicht, die auf den Träger laminiert ist und die die Klebstoffzusammensetzung nach einem der Ansprüche 1 bis 8 enthält.

**Revendications**

1. Composition adhésive comportant :

une structure noyau-enveloppe incluant une portion de noyau contenant un ingrédient actif, et une portion d'enveloppe recouvrant au moins une partie d'une surface de la portion de noyau et contenant un tensioactif ;
un premier élastomère ; et
un second élastomère différent du premier élastomère,
la valeur HLB du tensioactif étant de 4 ou plus et de 14 ou moins,
le tensioactif incluant au moins un tensioactif choisi parmi le groupe constitué d'un ester d'acide gras et de sorbitane, d'un ester d'acide gras et de glycérol, d'un ester d'acide gras et de propylène glycol, et d'un alcanolamide d'acide gras,
un groupe hydrocarbure du tensioactif incluant un groupe hydrocarbure saturé possédant 7 à 15 atomes de carbone ou un groupe hydrocarbure insaturé possédant 7 à 17 atomes de carbone,
la composition adhésive ayant un rapport pondéral de l'ingrédient actif sur le tensioactif (ingrédient actif : tensioactif) qui peut être de 1:0,5 à 1:2, et
la composition adhésive ayant une différence entre une valeur SP du premier élastomère et une valeur SP du second élastomère de 0,1 ou plus et de 3,0 ou moins,
dans laquelle la valeur SP est déterminée selon la méthode de Fedors telle que décrite dans la description.

2. Composition adhésive selon la revendication 1, dans laquelle le premier élastomère et le second élastomère ont chacun une valeur SP de 8 ou plus et de 11 ou moins,
dans laquelle la valeur SP est déterminée selon la méthode de Fedors telle que décrite dans la description.

3. Composition adhésive selon la revendication 1 ou 2, dans laquelle un mélange du premier élastomère et du second élastomère a un trouble mesuré conformément à la norme JIS K 7361 de 3 ou plus et de 75 ou moins.

4. Composition adhésive selon l'une quelconque des revendications 1 à 3, ayant un rapport pondéral du second élastomère sur le premier élastomère (second élastomère/premier élastomère) de 0,1 ou plus et de 10 ou moins.

5. Composition adhésive selon l'une quelconque des revendications 1 à 4, dans laquelle le premier élastomère est un élastomère acrylique.

6. Composition adhésive selon la revendication 5, dans laquelle le second élastomère est un élastomère acrylique différent.

7. Composition adhésive selon l'une quelconque des revendications 1 à 6, dans laquelle le tensioactif inclut au moins un tensioactif choisi parmi le groupe constitué d'un ester d'acide gras et de sorbitane, d'un ester d'acide gras et de glycérol, et d'un ester d'acide gras et de propylène glycol.

8. Composition adhésive selon l'une quelconque des revendications 1 à 7, dans laquelle l'ester d'acide gras et de glycérol est au moins un ester choisi parmi le groupe constitué d'un ester d'acide gras et de monoglycérol, d'un ester d'acide gras et de diglycérol, et d'un ester d'acide gras et de triglycérol.

9. Timbre adhésif comportant :

un support ; et
une couche d'adhésif qui est stratifiée sur le support et qui contient la composition adhésive selon l'une quelconque des revendications 1 à 8.

[FIG. 1.]

[FIG. 2.]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017014306 A
- JP 2014172840 A
- JP 2017154989 A

**Non-patent literature cited in the description**

- *Journal of the Adhesion Society of Japan,* 1986, vol. 22, 566